# EUROPÄISCHE PATENTSCHRIFT

(11) **EP 3 624 738 B1**
(45) Veröffentlichungstag und Bekanntmachung des Hinweises auf die Patenterteilung: **04.05.2022**
(21) Anmeldenummer: 18713825.0
(22) Anmeldetag: 16.03.2018
(51) Int. Cl.: A61F 2/60, A61F 2/68, A61F 2/70, A61F 2/76

(54) **VERFAHREN ZUR ERFASSUNG VON SENSORDATEN**
METHOD FOR CAPTURING SENSOR DATA
PROCÉDÉ D'ACQUISITION DE DONNÉES DE CAPTEUR

(30) Priorität: 17.05.2017 DE 102017110761
(43) Veröffentlichungstag der Anmeldung: 25.03.2020
(60) Teilanmeldung: 20195077.1
(73) Patentinhaber: Ottobock SE & Co. KGaA, 37115 Duderstadt (DE)
(72) Erfinder: ALBRECHT-LAATSCH, Erik, 37124 Rosdorf (DE); WILL, Christian, 37079 Göttingen (DE); DRIESEBERG, Jens, 99976 Duenwald (DE); BARTELS-SELLERING, Christopher, 37073 Göttingen (DE); NOLTE, Michael, 37136 Seeburg (DE); SZUFNAROWSKI, Filip, 37115 Duderstadt (DE); BORNMANN, Jonas, 37115 Duderstadt (DE)
(74) Vertreter: Gramm, Lins & Partner Patent- und Rechtsanwälte PartGmbB
(86) Internationale Anmeldenummer: PCT/EP2018/056754
(87) Internationale Veröffentlichungsnummer: WO 2018/210469

(56) Entgegenhaltungen:
- WO-A1-2015/110298
- WO-A1-2015/110298
- US-A1- 2008 164 979
- US-A1- 2008 164 979
- US-A1- 2014 145 848
- US-A1- 2014 145 848

## Beschreibung

Die Erfindung betrifft ein Verfahren, bei dem wenigstens zwei Sensoren an unterschiedlichen Körpersegmenten eines Menschen befestigt werden. Sie betrifft zudem ein System zum Durchführen eines derartigen Verfahrens.

Die Patentschrift US2008164979 A1 wird diesbezüglich als nächstliegender Stand der Technik angesehen.

Der Aufbau einer Prothese, insbesondere einer Beinprothese, muss sorgfältig ausgeführt und insbesondere individuell an den jeweiligen Patienten angepasst werden. Dies gilt sowohl für Oberschenkelamputierte (transfemoral), aber auch für Unterschenkelamputierte (transtibial), die noch über ein natürliches Kniegelenk an dem zu versorgenden Bein verfügen. Selbstverständlich gilt dies auch für alle anderen Prothesen. Der Prothesenaufbau muss dabei so erfolgen, dass beispielsweise ein möglichst natürliches Gangbild erzeugt wird, so dass der Träger der Prothese keine unbequemen, ungewohnten oder unnatürlichen Bewegungen machen muss, um die Prothese zu steuern. Gleichzeitig muss gewährleistet werden, dass in möglichst vielen Situationen eine möglichst hohe Sicherheit durch die Prothese an ihren Träger vermittelt wird.

Herkömmlicherweise wird ein statischer Aufbau, der an einem stehenden Patienten durchgeführt wird, und ein dynamischer Prothesenaufbau während des Gehens des Patienten durchgeführt. Während für den statischen Aufbau Systeme und Plattformen vorhanden sind, die dem Orthopädietechniker, der den Aufbau auch vornimmt, objektive Messdaten zur Verfügung stellen, wird der dynamische Aufbau oftmals noch auf der Basis von sehr subjektiven Daten, die der zuständige Orthopädietechniker beispielsweise beim Anschauen des Gangbildes für sich ermittelt, vorgenommen. Dies ist insbesondere dann von Nachteil, wenn beispielsweise aufgrund räumlicher Enge der Orthopädietechniker die nötigen Perspektiven nicht einnehmen kann und beispielsweise den Patienten nicht während des Gehens seitlich betrachten kann.

Um die Qualität des dynamischen Prothesenaufbaus zu verbessern, wäre es von Vorteil, ein System bereitstellen zu können, bei dem unterschiedliche Sensoren, die die benötigen Messwerte aufnehmen können, an unterschiedlichen Stellen des Körpers des Patienten und/oder an unterschiedlichen Positionen der Prothese befestigt werden könnten. Die entsprechenden Sensoren könnten beispielsweise Inertialdaten, wie beispielsweise Absolutwinkel oder Relativwinkel, beispielsweise einen Kniewinkel, Beschleunigungen oder Geschwindigkeit messen, die objektiv Auskunft über das Gangbild und den vorhandenen Prothesenaufbau liefern. Um verlässliche Daten zu generieren ist es jedoch notwendig, die Orientierung des Sensors am Körper oder der Prothese möglichst exakt zu kennen und dafür zu sorgen, dass sich diese Orientierung und/oder Ausrichtung während des Gehens und der dynamischen Analyse möglichst wenig, vorzugsweise gar nicht, ändert.

Aus der DE 10 2008 024 746 A1 ist eine orthopädietechnische Einrichtung für die unteren Extremitäten bekannt, bei der genau diese Orientierung und Ausrichtung eines Sensors an der Prothese bestimmt werden soll. Dazu muss das System zunächst in einen Lernmodus gebracht werden, in dem bestimmte Daten aufgenommen und so ausgewertet werden können, dass gegebenenfalls vorhandene Fehler und Ungenauigkeiten in der Ausrichtung und/oder Positionierung der Sensoren erkannt und aus den ermittelten Messdaten herausgerechnet werden können. Erst danach kann in den eigentlichen Betriebsmodus geschaltet werden, in dem die Sensoren die benötigten Werte ermitteln.

Eine Überprüfung der Orientierung und/oder Ausrichtung der Sensoren ist auf diese Weise jedoch nicht möglich, sofern nicht zu unterschiedlichen Zeitpunkten erneut in den Lernmodus geschaltet wird. Dies ist aufwendig und wird in der Praxis nicht durchgeführt.

Der Erfindung liegt daher die Aufgabe zugrunde, ein Verfahren vorzuschlagen, durch das ein dynamischer Prothesenaufbau einfach, verlässlich und objektiv durchgeführt werden kann.

Die Erfindung löst die gestellte Aufgabe durch ein Verfahren, bei dem wenigstens zwei Sensoren an unterschiedlichen Körpersegmenten eines Menschen befestigt werden, die wenigstens zwei Sensoren Signale, die Messdaten und eine individuelle Sensorkennung enthalten, an eine elektronische Datenverarbeitungseinrichtung senden und die elektronische Datenverarbeitungseinrichtung die Körpersegmente bestimmt, an denen die Sensoren angeordnet sind oder anzuordnen sind und die Messdaten der wenigstens zwei Sensoren den Körpersegmenten zuordnet. Diese Zuordnung erfolgt vorzugsweise automatisch. Dazu erkennt vorzugsweise die elektronische Datenverarbeitungseinrichtung die individuelle Sensorkennung.

Unter einem Körpersegment wird vorliegend sowohl ein natürliches Körperteil eines Menschen als auch ein Bauteil einer Orthese oder Prothese verstanden, die der Mensch trägt.

Durch das Verfahren werden die von dem Sensor ausgesandten Signale, die insbesondere die Messdaten enthalten, dem richtigen Körpersegment, an dem sich der Sensor befindet, zugeordnet. Auf diese Weise wird eine objektive Messwerterfassung erreicht, die einen objektiven dynamischen Prothesenaufbau erlaubt und so zu einer besseren Qualität der verschiedenen Prothesen führen wird. Zudem kann der wenigstens eine Sensor unterschiedlichen Körpersegmenten zugeordnet werden, was durch die elektronische Datenverarbeitungseinrichtung erkannt oder ermittelt werden kann, so dass der jeweilige Sensor bei unterschiedlichen Patienten an unterschiedlichen Körpersegmenten verwendet werden kann. Insbesondere für den Fall, dass mehrere Sensoren an unterschiedlichen Körpersegmenten anzuordnen sind, entfällt dadurch einerseits die Notwendigkeit, eine große Anzahl von Sensoren für die einzelnen Körpersegmente vorzuhalten, und andererseits ein kompliziertes und aufwendiges Verfahren, mit dem sichergestellt werden müsste, dass ein entsprechender Sensor immer an dem dafür vorgesehenen Körpersegment befestigt wird. Dies ist einerseits für den Orthopädietechniker aufwendig und unpraktisch und andererseits fehleranfällig. Diese Nachteile werden durch das erfindungsgemäße Verfahren vermieden.

In einer bevorzugten Ausgestaltung senden die wenigstens zwei Sensoren die individuelle Sensorkennung erst nach Aufforderung. Diese Aufforderung erfolgt vorzugsweise durch ein entsprechendes Steuersignal, das von der elektronischen Datenverarbeitungseinrichtung ausgesandt und von dem Sensor detektiert wird. Werden mehrere Sensoren verwendet, enthält das Steuersignal vorzugsweise Informationen darüber, welcher Sensor angesprochen wird und seine individuelle Sensorkennung übermitteln soll.

Alternativ kann der Sensor auch eine Betätigungseinrichtung, beispielsweise einen Druckknopf aufweisen. Wenn die Betätigungseinrichtung betätigt wird, sendet der Sensor seine individuelle Sensorkennung, die von der elektronischen Datenverarbeitungseinrichtung detektiert wird.

In einer bevorzugten Ausgestaltung des Verfahrens ist der elektronischen Datenverarbeitungseinrichtung wenigstens ein Detektor zugeordnet, der zumindest einen Teil der Signale des Sensors detektiert, wobei die elektronische Datenverarbeitungseinrichtung aus den detektierten Signalen, insbesondere aus einer Richtung oder Lage, aus der die individuelle Sondererkennung gesendet wird, oder aus einem zeitlichen Verlauf der Messdaten, das Körpersegment bestimmt.

Die Sensorkennung kann beispielsweise über sichtbares Licht ausgesandt werden. Dies kann vorteilhafterweise über wenigstens eine LED, vorzugsweise mehrere LEDs, die besonders vorzugsweise Licht unterschiedlicher Farben aussenden, geschehen. Die individuelle Sensorkennung kann dabei Kombinationen aus Blinkfrequenz, Farben, Intensität und/oder Muster, eine bestimmte Reihenfolge oder einen Code aus einer oder mehreren dieser Parameter enthalten. Wird die individuelle Sensorkennung von einem Sensor ausgesandt, kann sie von einem entsprechenden Detektor, der der Datenverarbeitungseinrichtung zugeordnet ist und im genannten Ausführungsbeispiel eine Kamera sein kann, erfasst. Selbstverständlich sind auch andere lichtsensitive Sensoren denkbar. Alternativ oder zusätzlich dazu kann die Sensorkennung auch nicht sichtbare elektromagnetische Strahlung, beispielsweise im Infrarotbereich, und/oder hörbare und/oder nicht hörbare akustische Signale, beispielsweise im Ultraschallbereich, enthalten. Entsprechend muss der Detektor in der Lage sein, zumindest einen Teil dieser individuellen Sensorkennung zu detektieren, der es der elektronischen Datenverarbeitungseinrichtung erlaubt, den Sensor anhand der detektierten Teile der individuellen Sensorkennung zu identifizieren. Vorteilhafterweise ist der Detektor in der Lage, die vollständige Sensorkennung zu detektieren.

Der Detektor, der der Datenverarbeitungseinrichtung zugeordnet ist, kann jedoch vorteilhafterweise nicht nur die individuelle Sensorkennung, sondern auch weitere Informationen detektieren, aus denen sich die Position des sendenden Sensors ergibt. Dies kann beispielsweise die Richtung sein, aus der die Senderkennung gesendet wird, so dass die elektronische Datenverarbeitungseinrichtung in der Lage ist, das Körpersegment zu bestimmen, an dem der Sensor angeordnet ist. Anschließend weist die elektronische Datenverarbeitungseinrichtung die individuelle Sensorkennung des sendenden Sensors vorzugsweise dem jeweiligen Körpersegment zu, so dass bei der Verwendung des Sensors auch die gesendeten Messdaten als von diesem Sensor kommend identifiziert und dem jeweiligen Körpersegment zugeordnet werden können.

Vorteilhafterweise wird die individuelle Sensorkennung dem jeweiligen Sensor übermittelt, bevor er an dem Körpersegment befestigt wird. Alternativ dazu kann die Kennung auch an den Sender übermittelt werden, wenn dieser bereits an einem Körpersegment des Menschen befestigt wurde. Der Sensor empfängt seine individuelle Kennung vorteilhafterweise von einer dafür mit der elektronischen Datenverarbeitungseinrichtung verbundenen Sendeeinrichtung und kann vorzugsweise über ein geeignetes Kommunikationsprotokoll der elektronischen Datenverarbeitungseinrichtung eine Bestätigung übermitteln. Aus dieser Bestätigung, die vorteilhafterweise die individuelle Sensorkennung enthält, oder aus den ansonsten übermittelten Signalen kann die elektronische Datenverarbeitungseinrichtung vorteilhafterweise auf die Position und/oder die Richtung, in der sich der Sensor befindet, und damit auf das Körpersegment des Menschen zurückschließen.

Alternativ oder zusätzlich zu diesem Vorgehen kann die elektronische Datenverarbeitungseinrichtung auch aus den detektierten Signalen, beispielswiese dem zeitlichen Verlauf der Messdaten, auf das Körpersegment schließen, an dem sich der Sensor befindet. Dazu ist es von Vorteil, wenn in der elektronischen Datenverarbeitungseinrichtung hinterlegt ist, zu welchem Bewegungsablauf, beispielweise Gehen in der Ebene, Gehen auf schräger Ebene, Treppensteigen, Hinsetzen, Laufen oder Aufstehen, die detektierten zeitlichen Verläufe der Messdaten gehören. Durch Vergleich mit hinterlegten zeitlichen Verläufen von Messdaten kann die elektronische Datenverarbeitungseinrichtung erkennen, an welchem Körpersegment sich der Sensor, zu dem diese detektierten Messdaten gehören, befindet.

Alternativ oder zusätzlich zum Vergleich mit hinterlegten zeitlichen Verläufen von Messdaten können auch andere Klassifikationsalgorithmen verwendet werden. Dies kann beispielsweise ein simples Einsortieren nach Schwellwerten sein oder ein komplexes, selbstständig entscheidendes neurales Netz beinhalten.

Vorzugsweise enthalten die Signale des Sensors zusätzlich Informationen über das Körpersegment, an dem der Sensor befestigt ist. Diese können auf unterschiedlichste Weise eingestellt oder ermittelt werden. In einer besonders bevorzugten Ausgestaltung verfügt der Sensor oder ein Gehäuse, in dem sich der Sensor befindet, über eine Einstelleinrichtung, beispielsweise einen Dreh- oder Schieberegler. Durch Einstellen dieser Einstelleinrichtung werden die in den Signalen des Sensors enthaltenen Informationen über das Körpersegment codiert. Die einzelnen Einstellmöglichkeiten beispielsweise eines Schiebe- oder Drehreglers sind den verschiedenen Körpersegmenten des Menschen zugeordnet, so dass die Person, die den Sensor an dem Körpersegment befestigt hat oder befestigt, die Einstelleinrichtung entsprechend einstellt. Alternativ oder zusätzlich dazu kann der Sensor mittels einer Halterung an dem Körpersegment befestigt sein, die die vom Sensor gesendeten Signale beeinflusst. Die Halterung ist vorzugsweise an das jeweilige Körpersegment des Menschen angepasst. Eine Halterung für einen Sensor, der beispielsweise am Rumpf des Menschen angeordnet werden soll, ist naturgemäß anders ausgestaltet als eine Halterung für einen Sensor, der am Oberarm, am Handgelenk oder am Knie angeordnet werden soll. Da unterschiedliche Halterungen für die unterschiedlichen Körpersegmente verwendet werden müssen, kann in der Halterung beispielweise über einen elektrischen Schaltkreis oder einen RFID-Chip das vom Sensor ausgesandte Signal bezüglich des Körpersegments angepasst werden. Gegebenenfalls ist es von Vorteil, in diesem Fall an der Halterung einen Schalter oder eine Einstelleinrichtung vorzusehen, mit der codiert werden kann, ob die Halterung an einem linken oder einem rechten Körpersegment angeordnet ist.

In einer besonders einfachen Ausgestaltung kann der Sensor die Informationen über das Körpersegment der jeweiligen Halterung entnehmen.

Bevorzugt ermittelt die Halterung das Körpersegment, an dem sie befestigt ist. Dies kann beispielsweise über eine Umfangmessung des Körpersegments geschehen. Verfügt die Halterung beispielsweise über einen ausziehbaren oder in der Länge verstellbaren Gurt, kann aus der Länge des Gurtes, die benötigt wird, um das Körpersegment zu umgreifen und/oder zu umschließen, ermittelt werden, um welche Art Körpersegment es sich handelt. In diesem Fall müsste beispielsweise lediglich zusätzlich festgelegt werden, ob es sich, sofern das Körpersegment mehrfach vorhanden ist, um eine rechte oder eine linke Ausführung des Körpersegments, beispielsweise eines Beines oder eines Armes, handelt. Alternativ dazu können aufgedruckte Barcodes oder sonstige Codes, Orientierungsmarker, Glyphen oder sonstige Elemente verwendet werden.

Bevorzugt ist die Halterung eingerichtet, die Art des Sensors zu erkennen, der an ihr befestigt ist. So können Informationen über Körpersegmente und/oder Sensoren von der Halterung ermittelt werden und entweder von der Halterung selbst an die elektronische Datenverarbeitungseinrichtung gesandt werden oder über den Sensor versendet werden.

Vorzugsweise verfügt der Sensor über eine Einstelleinrichtung, durch die die Informationen über das Körpersegment eingestellt werden. Damit kann es sich beispielweise um ein manuell betätigbares Element, beispielsweise einen Dreh- oder Schieberegler handeln. Je nach Körpersegment, an dem der Sensor angeordnet wird, werden diese Einstellelemente in eine bestimmte Position und/oder Stellung gebracht, wodurch die Informationen über das Körpersegment, die diese Lage des Sensors enthalten, eingestellt werden.

Vorzugsweise senden die wenigstens zwei Sensoren die Signale nur an die elektronische Datenverarbeitungseinrichtung, wenn sie ein entsprechendes Abfragesignal erhalten haben. Auf diese Weise kann sichergestellt werden, dass die elektronische Datenverarbeitungseinrichtung nur Signale eines einzelnen Sensors empfängt und nicht mehrere Signale unterschiedlicher Sensoren, die an unterschiedlichen Körpersegmenten des Menschen befestigt sind, einander überlagern und gegebenenfalls nicht ausgewertet werden können.

Erfindungsgemäß sind diese wenigstens zwei Sensoren eingerichtet, Informationen über Abstände zwischen jeweils zwei dieser Sensoren zu ermitteln und diese in den gesendeten Signalen an die elektronische Datenverarbeitungseinrichtung zu senden. In diesem Fall kann die elektronische Datenverarbeitungseinrichtung aus hinterlegten Mustern von Abständen über die einzelnen Positionen und Körpersegment, an denen die Sensoren befestigt sind, schließen.

Um die Informationen über Abstände zwischen zwei Sensoren zu ermitteln, ist es von Vorteil, wenn zumindest einer, vorzugsweise alle der Sensoren in der Lage sind, entsprechende Testsignale, beispielswiese in Form von Vibrationen, elektromagnetischer Strahlung oder Schall, auszusenden, die von den jeweils anderen Sensoren detektiert werden können. Es hat sich als vorteilhaft herausgestellt, wenn die Sensoren, die entsprechende Testsignale empfangen, ein entsprechendes Antwortsignal aussenden. Über Laufzeiten des Testsignals und des entsprechenden Antwortsignals kann auf Abstände der verschiedenen Sensoren untereinander und damit auf eine Anordnung der Sensoren relativ zueinander geschlossen werden. Da die möglichen Körpersegmente, an denen Sensoren befestigt werden können, bekannt sind, kann aus diesen Informationen ermittelt werden, welcher Sensor an welchem Körpersegment angeordnet ist. Wird zudem das Testsignal vom ursprünglich sendenden Sensor in eine bestimmte Richtung oder in einen bestimmten Raumbereich gerichtet ausgesandt, kann auch zwischen rechten und linken Körpersegmenten, beispielsweise Beinen oder Armen, unterschieden werden.

Alternativ oder zusätzlich dazu können die Sensoren Testsignale aussenden, die von anderen Sensoren detektiert werden. Informationen über die so detektierten Signale, insbesondere die Detektionszeitpunkte, können dann entweder von den detektierenden Sensoren an die elektronische Datenverarbeitungseinrichtung übermittelt werden oder in Form von Antwortsignalen an den aussendenden Sensor übermittelt werden. Bevorzugt werden sie alternativ oder zusätzlich dazu von der elektronischen Datenverarbeitungseinrichtung empfangen.

Vorteilhafterweise können die Sensoren einen Abstand von einem anderen Gegenstand, beispielsweise dem Boden, bestimmen. Dies kann beispielsweise über Ultraschallsignale erfolgen, die in Richtung des Bodens ausgesandt werden. Die reflektierten Ultraschallsignale können detektiert werden und aus der daraus zu bestimmenden Laufzeit und der bekannten Schallgeschwindigkeit auf den Abstand vom Boden geschlossen werden. Auf diese Weise kann beispielsweise festgestellt werden, ob ein Sensor an einem Knie, einem Oberschenkel oder einer Schulter angeordnet ist. Selbstverständlich können auch andere Körpersegment ermittelt werden, solange diese unterschiedliche Abstände vom Boden aufweisen. Alternativ oder zusätzlich dazu kann auch ein präzise arbeitendes Positionierungssystem, beispielweise ein Global Positioning System (GPS), verwendet werden, um die Position der einzelnen Sensoren möglichst exakt zu bestimmen und so festzustellen, an welchem Körpersegment die Sensoren angeordnet sind.

Zur Bestimmung von Positionen der einzelnen Sensoren relativ zueinander oder relativ zu einem bestimmten Punkt am Körper des Trägers können auch Körpersignale, die vom Menschen ausgehen, verwendet werden. So können beispielsweise der Pulsschlag, die Herzfrequenz, der entsprechende Blutdruck und/oder das EKG verwendet werden, um beispielsweise einen Abstand des Sensors vom Herzen des Menschen zu bestimmen. Selbstverständlich sind alle anderen Biosignale, die vom Körper ausgesandt werden, und eine Orts- und/oder Abstandsbestimmung erlaubt, ebenfalls allein oder in Kombination mit anderen hier aufgeführten Signalen verwendbar.

Die Erfindung löst die gestellte Aufgabe zudem durch ein Verfahren, bei dem wenigstens zwei Sensoren an einem Körpersegment eines Menschen befestigt werden, die wenigstens zwei Sensoren Signale, die Messdaten enthalten, an eine elektronische Datenverarbeitungseinrichtung senden und die elektronische Datenverarbeitungseinrichtung ein Steuersignal an die wenigstens zwei Sensor aussendet und die wenigstens zwei Sensor als Reaktion auf das Steuersignal ein von einem Menschen detektierbares Antwortsignal aussenden. Dieses Antwortsignal, beispielsweise ein Audiosignal, ein haptisches Signal und/oder ein optisches Signal, wird vom Menschen, beispielsweise einem Orthopädietechniker detektiert, der anschließend vorzugsweise die Position des entsprechenden Sensors der elektronischen Datenverarbeitungseinrichtung mitteilt. Dazu verfügt diese über eine Schnittstelle, durch die ein Mensch entsprechende Daten in die Datenverarbeitungseinrichtung eingeben kann.

Die Erfindung löst die gestellte Aufgabe zudem durch ein System mit wenigstens zwei Sensoren und einer elektronischen Datenverarbeitungseinrichtung, das eingerichtet ist zum Durchführen eines derartigen Verfahrens.

Die wenigstens zwei Sensoren müssen natürlich an einer Prothese oder Orthese befestigt werden. Dann bilden diese Elemente vorzugsweise ein System mit einer Orthese und/oder einer Prothese und einer Halterung zum Befestigen eines Sensors an einem Gegenstand, wobei die Halterung wenigstens zwei Befestigungselemente zum Befestigen der Halterung an dem Gegenstand, die voneinander durch wenigstens ein Abstandselement beabstandet sind, und wenigstens ein Sensorbefestigungselement zum Befestigen des Sensors an der Halterung aufweist, wobei das wenigstens eine Abstandselement in einer ersten Richtung eine kleinere Biegesteifigkeit aufweist als in einer zweiten Richtung, die senkrecht auf der ersten Richtung steht.

Ein solches System stellt eine eigenständige Erfindung dar oder kann in Kombination mit anderen hier beschriebenen Merkmalen verwendet werden. Dies gilt auch für eine Halterung für ein solches System.

Herkömmlicherweise werden Sensoren beispielsweise über einen Gurt, der um ein Körperteil oder ein Prothesenteil herumgelegt wird, an dem jeweiligen Körperteil oder Prothesenteil befestigt. Dabei sind oftmals Verdrehungen des Sensors relativ zum Körperteil, beispielsweise einem Oberschenkel, möglich, die zur optimalen Auswertung der vom Sensor ermittelten Messdaten erkannt und auch quantifiziert werden müssen. Zudem kann es durch Erschütterungen und Beschleunigungen, die beim Gehen auftreten, zu Verschiebungen oder zum Verrutschen der Sensoren relativ zum Körperteil oder zum Prothesenteil kommen.

Unter einem Körpersegment wird vorliegend sowohl ein natürliches Körperteil eines Menschen als auch ein Bauteil einer Orthese oder Prothese verstanden, die der Mensch trägt.

Durch die erfindungsgemäß vorgesehenen zwei Befestigungselemente, die beispielsweise in Form von zwei Gurten vorliegen können, die um das jeweilige Körperteil oder Prothesenteil herum gelegt werden, wird die Orientierung des zwischen den beiden Befestigungselementen vorhandenen Abstandselementes festgelegt. Eine Rotation oder ein Verschieben dieses Elementes relativ zum Körperteil oder Prothesenteil, beispielsweise relativ zum Oberschenkel, ist nur noch in einem sehr begrenzten Rahmen, vorzugsweise gar nicht mehr möglich. Damit ist die Orientierung zumindest des Abstandselementes relativ zum Gegenstand, also insbesondere relativ zum Körperteil oder zum Prothesenteil, festgelegt und kann nicht oder nur unwesentlich geändert werden. Der Sensor selbst wird über das Sensorbefestigungselement an der Halterung, besonders bevorzugt am Abstandselement, befestigt. Durch beispielsweise am Sensor oder am Sensorgehäuse, in dem sich der Sensor befindet, vorgesehenen Markierungen lässt sich eine relative Orientierung des Sensorgehäuses oder des Sensors relativ zum Abstandselement leicht ablesen und vorteilhafterweise einstellen. Auf diese Weise kann bereits das Befestigen des Sensors am Gegenstand, also vorzugsweise am Körperteil oder am Prothesenteil, die Ausrichtung und Orientierung des Sensors relativ zu diesem Gegenstand bestimmt und festgelegt werden.

Durch die spezielle Ausgestaltung des Abstandselementes mit der anisotropen Biegesteifigkeit kann gewährleistet werden, dass eine für den Sensor optimale Orientierung beibehalten wird und dennoch eine Bewegung in einer anderen Richtung möglich ist. Ein Sensor kann beispielweise ein Beschleunigungssensor oder ein Winkelsensor sein, der mit einer vorbekannten am Sensor ortsfest vorhandenen Längsachse beispielsweise parallel zum Gegenstand, beispielsweise einem Oberschenkel, ausgerichtet werden sollte, um optimale Messdaten zu erhalten. Dies ist mit einer erfindungsgemäßen Halterung einfach möglich. Die beiden Befestigungselemente werden an zwei voneinander durch das Abstandselement beabstandeten Positionen am Oberschenkel festgelegt. Dabei kann sichergestellt werden, dass sich das Abstandselement parallel zum Oberschenkel, beispielsweise zum Oberschenkelknochen, erstreckt. Der Sensor, der bevorzugt bereits an der Halterung befestigt ist oder nachträglich an ihr befestigt werden kann, hat daher eine vorbekannte vorzugsweise einstellbare Ausrichtung relativ zum Abstandselement und damit relativ zum Oberschenkel. Die gedachte Längsachse des Oberschenkels und die entsprechende festzustellende Richtung des Sensors verlaufen parallel und spannen daher eine Ebene auf. Das Abstandselement verfügt nun in zwei unterschiedlichen Richtungen über unterschiedliche Biegesteifigkeiten, die vorteilhafterweise so gelegt sind, dass die Biegesteifigkeit besonders hoch ist für Biegungen des Abstandselementes, die die vorbestimmte Sensorrichtung aus der gemeinsam mit der Symmetrieachse des Oberschenkels aufgespannten Ebene herausbewegt, so dass eine solche Bewegung, die die Orientierung verändern und damit die Qualität der Messdaten reduzieren würde, nur schwer oder vorzugsweise gar nicht möglich ist. In einer Richtung senkrecht dazu, die vortielhafterweise die vorbestimmte Richtung des Sensors nicht aus der gemeinsam mit der Symmetrieachse des Oberschenkels aufgespannten Ebene herausführt, ist hingegen deutlich leichter möglich, da hier die Biegesteifigkeit reduziert ist.

Dies ist selbstverständlich nicht auf Ausführungsbeispiele begrenzt, bei denen ein Sensor am Oberschenkel einer Person befestigt wird.

Vorzugsweise ist das wenigstens eine Abstandselement ein Druckkraftübertragungselement. Damit ist sichergestellt, dass die beiden Befestigungselemente immer den gleichen Abstand aufweisen und das Abstandselement drucksteif ist.

In einer bevorzugten Ausgestaltung verfügt das wenigstens eine Abstandselement über wenigstens zwei nebeneinander verlaufende Einzelelemente, insbesondere Stege, Spangen, Stäbe oder Rohre. Auf diese Weise ist es besonders einfach möglich, die unterschiedlichen Biegesteifigkeiten zu erreichen, da die Einzelelemente selbst eine isotrope, also in alle Richtungen gleiche Biegesteifigkeit aufweisen können und allein durch die Positionierung der unterschiedlichen Einzelelemente nebeneinander die gewünschten unterschiedlichen Biegesteifigkeiten des Abstandselementes erreicht werden.

Als besonders vorteilhaft hat sich herausgestellt, wenn die Einzelelemente einstückig miteinander ausgebildet sind. Besonders vorzugsweise ist das gesamte Abstandselement, besonders bevorzugt die gesamte Halterung einstückig ausgebildet. Sie kann beispielsweise ein Kunststoffelement sein, das beispielsweise aus einer Kunststoffplatte herausgeschnitten, herausgesägt oder herausgestanzt wurde. Alternativ können auch 3D-Druckbauteile oder metallische Bauteile verwendet werden. In einer bevorzugten Ausgestaltung sind die Einzelelemente aus einem Kunststoff oder einem Faserverbundwerkstoff, insbesondere einem Kohlefaserverbundwerkstoff oder einem Glasfaserverbundwerkstoff hergestellt. Dies ist insbesondere dann von Vorteil, wenn die Einzelelemente und das Abstandselement nicht einstückig ausgebildet sind. In diesem Fall können Kohlefaserstäbe oder Glasfaserstäbe verwendet werden, die leicht herzustellen sind, eine hohe Biegesteifigkeit aufweisen und dennoch über ein geringes Eigengewicht verfügen.

Vorzugsweise ist das wenigstens eine Sensorbefestigungselement an dem wenigstens einen Abstandselement angeordnet. Als besonders bevorzugt hat sich dabei herausgestellt, wenn das Sensorbefestigungselement in der Mitte zwischen den beiden Befestigungselementen angeordnet ist.

Vorteilhafterweise ist das Sensorbefestigungselement lösbar an dem wenigstens einen Abstandselement angeordnet, bevorzugt angeklemmt oder aufgeklemmt. Auf diese Weise lässt sich beispielsweise besonders leicht der verwendete Sensor austauschen, sofern dies aufgrund der benötigten Messdaten oder zur Wartung und/oder Reparatur des Sensors notwendig ist. Zudem lässt sich erreichen, dass das Sensorbefestigungselement entlang der Längserstreckung des Abstandselementes verschiebbar angeordnet sein kann, so dass auch die Position des Sensors am Gegenstand, also beispielsweise am Körperteil oder am Prothesenteil, nachträglich einstellbar ist und auf die optimale Position gebracht werden kann. Gleichzeitig wird dadurch die Orientierung des wenigstens einen Sensors nicht beeinflusst.

Vorzugsweise verfügt der Gegenstand über eine Längsrichtung und ist insbesondere ein Prothesenbauteil, ein Orthesenbauteil oder ein Segment eines menschlichen Körpers. Vorzugsweise ist die Halterung derart ausgebildet, dass eine Richtung, in der die wenigstens zwei Befestigungselemente voneinander beabstandet sind, der Längsrichtung entspricht oder parallel zu Ihr verläuft, wenn die Halterung an dem Gegenstand angeordnet ist. Diese Richtung entspricht vorteilhafterweise einer Erstreckungsrichtung des Abstandselementes. Wird die Halterung beispielsweise an einem Prothesenbauteil oder einem Segment eines menschlichen Körpers angeordnet, ist auf diese Weise gewährleitet, dass die Orientierung der Halterung und damit vorzugsweise auch die Orientierung des Sensors relativ zu dem Gegenstand festgelegt ist, so dass fehlerhafte Bedienungen und daraus resultierende ungenaue oder falsche Messwerte verhindert oder zumindest verringert werden.

Der wenigstens eine Sensor weist vorzugsweise einen Inertialsensor, einen insbesondere elektromagnetischen Raumlagesensor, einen Winkelsensor, einen Beschleunigungssensor, einen Sensor zum Detektieren von Biosignalen des menschlichen Körpers, beispielsweise EKG, Blutdruck, Puls oder ähnliches, und/oder einen elektromagnetischen Tracker auf.

Auch ein System aus einer derartigen Halterung sowie einem Sensor, der an dem wenigstens einen Sensorbefestigungselement befestigt ist, bildet eine eigenständige Erfindung, die auch in Kombination mit anderen hier beschriebenen Merkmalen verwendet werden kann. Vorteilhafterweise ist das wenigstens eine Sensorbefestigungselement und der wenigstens eine Sensor derart ausgebildet, dass der Sensor nur in wenigen, vorzugsweise zwei, besonders vorzugsweise nur einer Orientierung an dem Sensorbefestigungselement befestigbar ist. Auf diese Weise ist gewährleistet, dass der Sensor nur in bestimmten Orientierungen befestigbar ist, so dass die Orientierung des Sensors relativ zur Halterung und damit relativ zum Abstandselement oder zum Sensorbefestigungselement nicht detektiert, überprüft und gegebenenfalls eingestellt werden muss und zudem keine Quelle von Messfehlern oder sonstigen Ungenauigkeiten ist.

Soll der Prothesenaufbau dynamisch untersucht werden, ist es von Vorteil, Sensoren an unterschiedlichen Stellen und Körpersegmenten eines Menschen anordnen zu können. Eine elektronische Datenverarbeitungseinrichtung ist dann vorzugsweise in der Lage, Messdaten und Signale, die insbesondere auch eine individuelle Sensorkennung beinhalten, dem Körpersegment zuzuordnen, an dem der jeweilige Sensor befestigt ist.

Der Aufbau und/oder die Einrichtung einer Orthese und/oder Prothese ist für den Komfort und die Funktionalität von hoher Wichtigkeit. Daher ist es von Vorteil, die Einrichtung zu prüfen. Dies wird erreicht durch ein Verfahren zum Einrichten einer Prothese oder Orthese oder zum Bestimmen von Fehlstellungen im Aufbau einer Prothese oder Orthese der unteren Extremität mit den Schritten a) Aufnehmen von ersten Messdaten wenigstens eines Sensors, der an einem Körpersegment eines Menschen befestigt ist, wobei die ersten Messdaten einem ersten Bewegungszustand des Menschen zugeordnet werden, b) Aufnehmen von zweiten Messdaten wenigstens eines Sensors, der an einem Körpersegment des Menschen befestigt ist, wobei die zweiten Messdaten einem zweiten Bewegungszustand des Menschen zugeordnet werden und c) Auswerten der ersten und zweiten Messdaten.

Dieses Verfahren stellt eine eigene Erfindung dar oder kann in Kombination mit anderen hier beschriebenen Merkmalen verwendet werden.

Diesem Verfahren liegt die Erkenntnis zugrunde, dass es zum optimalen Prothesenaufbau oftmals notwendig ist, Messdaten von Sensoren in unterschiedlichen Bewegungszuständen aufzunehmen und gleichzeitig zu werten. Daher sind der erste Bewegungszustand und der zweite Bewegungszustand voneinander verschieden. Auf diese Weise werden Informationen erhalten, die durch das einfache Auswerten der Messdaten nur eines der beiden Bewegungszustände nicht erhalten werden können. Dabei ist selbstverständlich notwendig, dass die beiden Bewegungszustände voneinander verschieden sind.

Das Verfahren kann vollständig von einem Computer oder einer elektronischen Datenverarbeitungseinrichtung durchgeführt werden, sofern diese Zugriff auf die aufgenommenen ersten und zweiten Messdaten hat. Diese werden von dem jeweiligen wenigstens einem Sensor der elektronischen Datenverarbeitungseinrichtung übermittelt und in einem elektronischen Datenspeicher vorteilhafterweise abgespeichert.

In einer bevorzugten Ausgestaltung wird der zweite Bewegungszustand auf der Grundlage der ersten Messdaten ausgewählt. Vorzugsweise werden zunächst die ersten Messdaten ausgewertet. Dabei können bereits Indizien für vorhandene Fehlstellungen ermittelt werden, die durch Messungen in einem zweiten Bewegungszustand verifiziert, bestätigt oder wiederlegt werden können. Es ist daher oftmals von Vorteil, zunächst die ersten Messdaten auszuwählen um den optimalen zweiten Bewegungszustand auszuwählen, sodass mit den zweiten Messdaten die aus den ersten Messdaten noch vorhandenen Unklarheiten und/oder Mehrdeutigkeiten auflösen zu können.

Alternativ dazu ist es selbstverständlich auch möglich, dass einem ausgewählten ersten Bewegungszustand ein fester zweiter Bewegungszustand zugeordnet wird. Ist der erste Bewegungszustand beispielsweise das Treppensteigen nach oben, kann es sinnvoll sein, das Treppensteigen nach unten oder das Gehen auf einer schiefen Ebene, also einer Neigung nach oben oder unten, zuzuordnen. Durch eine derartig feste Zuordnung können eine Vielzahl unterschiedlicher Fehlstellungen aus den Messdaten ermittelt werden. Die feste Zuordnung ist dann sinnvoll, wenn einem ersten Bewegungszustand nur ein einziger zweiter Bewegungszustand zugeordnet werden kann. Sobald hier Mehrdeutigen auftreten und für unterschiedliche Auswerteergebnisse aus den Auswertungen der ersten Messdaten unterschiedliche zweite Bewegungszustände folgen können, ist eine derartige feste Zuordnung nicht mehr sinnvoll.

Für bestimmte Prothesen und/oder Patienten ist es zudem sinnvoll, mehr als zwei Bewegungszustände zu verwenden und in jedem dieser mehreren Bewegungszustände separate Messdaten aufzunehmen. In diesem Fall ist es von Vorteil, eine feste Reihenfolge der unterschiedlichen Bewegungszustände zu wählen und die jeweils aufgenommenen Messdaten dem jeweils aktuellen Bewegungszustand zuzuordnen.

Vorzugsweise wird der ausgewählte zweite Bewegungszustand über eine Kommunikationseinrichtung, insbesondere durch ein Audiosignal und/oder ein haptisches Signal und/oder ein visuelles Signal, angezeigt. Eine Vorrichtung, mit der das Verfahren durchgeführt werden kann, verfügt folglich über eine Kommunikationseinrichtung, beispielsweise ein Display oder eine Anzeigeeinrichtung, die beispielsweise in Form von unterschiedlich farbigen LEDs, Lampen oder sonstigen Anzeigeelementen vorliegen kann. Selbstverständlich kann auch ein Lautsprecher vorhanden sein, über den Audiosignale, beispielsweise gesprochene Worte, ausgesandt werden können. Vorzugsweise verfügt die Kommunikationseinrichtung zudem über ein Mikrofon, so dass eine Spracheingabe von Befehlen oder Anweisungen möglich ist. Nachdem die ersten Messdaten ausgewertet wurden und der zweite Bewegungszustand auf der Basis dieser Auswertung ermittelt wurde, wird einem Benutzer der Vorrichtung, beispielsweise einem Orthopädietechniker, über die Kommunikationseinrichtung mitgeteilt, welches der zweite Bewegungszustand ist. Der Patient wird dann beispielsweise durch die Kommunikationseinrichtung oder durch einen Orthopädietechniker aufgefordert, den nächsten Bewegungszustand einzunehmen und beispielsweise Treppen zu steigen, schneller oder langsamer zu gehen, sich hinzustellen oder hinzusetzen. Dabei ist jedoch zu beachten, dass das Verfahren unabhängig von der tatsächlichen Ausführung der unterschiedlichen Bewegungszustände durch den Menschen durchführbar ist. Die nach einer Aufforderung durch die Kommunikationseinrichtung ermittelten Messdaten werden unabhängig davon, ob der zweite Bewegungszustand tatsächlich eingenommen und zutreffend ausgeführt wurde oder nicht, dem zweiten Bewegungszustand zugeordnet. Wie bereits dargelegt, kann das Verfahren vollständig von einem Computer durchgeführt werden, der Zugriff auf die von dem wenigstens einen Sensor aufgenommenen Messdaten hat.

Vorteilhafterweise wird der erste Bewegungszustand aus den ersten Messdaten und/oder der zweite Bewegungszustand aus den zweiten Messdaten erkannt. Dies kann beispielsweise geschehen, in dem bestimmte Messdatenverläufe, beispielsweise ein Kniewinkelverlauf beim Gehen, in einem elektronischen Datenspeicher hinterlegt sind. Die aufgenommenen Messdaten werden anschließend mit einer Reihe dieser unterschiedlichen Bewegungsmuster und Datenverläufe verglichen bis eine Übereinstimmung gefunden wird. Der entsprechende Bewegungszustand wird als erster Bewegungszustand oder zweiter Bewegungszustand erkannt und entsprechend den Messdaten zugeordnet.

Vorzugsweise wird nach dem Auswerten der ersten und zweiten Messdaten eine Korrekturmaßnahme ermittelt und vorzugsweise mittels der Kommunikationseinrichtung ausgegeben. Diese Korrekturmaßnahme betrifft den Prothesenaufbau und kann beispielsweise in einer Verschiebung des Fußes relativ zum Unterschenkel und/oder zum Knie, oder einer anderen Positionierung der einzelnen Bauteile der Prothese relativ zueinander bestehen. Die Korrekturmaßnahme kann jedoch zumindest auch oder nur darin bestehen, ein verwendetes Bauteil in der Orthese oder der Prothese gegen ein anderes Bauteil auszutauschen und so beispielsweise einen Prothesenfuß, der in einer Beinprothese verwendet wird, gegen einen anderen Prothesenfuß auszutauschen. Damit kann gewährleistet werden, dass der Träger der Prothese oder Orthese nicht nur eine optimal aufgebaute Prothese oder Orthese, sondern auch eine Prothese oder Orthese aus einer möglichst optimalen Kombination einzelner Bauteile bekommt und verwenden kann.

Vorzugsweise wird die Korrekturmaßnahme durch die Kommunikationseinrichtung an ein Bauteil der Prothese oder Orthese übermittelt. Dies kann kabelgebunden oder kabellos, beispielsweise über Funk, WiFi oder Bluetooth erfolgen. Das Bauteil der Orthese oder Prothese ist vorzugsweise eingerichtet, die entsprechenden Signale umzusetzen und die darin enthaltene Korrekturmaßnahme als Reaktion auf die empfangenen Signale umzusetzen. Dies kann beispielsweise durch eine Erhöhung oder Reduzierung eines Beugewiderstands, eine Veränderung einer Position eines Bauteiles oder eine Einstellung einer Dämpfung geschehen. Sicherheitsrelevante Änderungen und/oder Korrekturmaßnahmen sind vorzugsweise durch einen Menschen, beispielsweise einen Orthopädietechniker zu bestätigen.

Die Korrekturmaßnahme ist vorzugsweise möglichst konkret, so dass sie von einem Orthopädietechniker leicht umgesetzt werden kann. Sie kann beispielsweise Anweisungen enthalten welche Schraube oder welcher Einstellmechanismus wie weit in welche Richtung gedreht oder in welcher Weise eingestellt werden soll.

Zusätzlich oder alternativ zu einer Korrekturmaßnahme kann auch eine Trainingsempfehlung ausgegeben werden, um dem Menschen den optimalen Umgang mit der Prothese oder Orthese zu vermitteln.

Vorzugsweise werden bei dem Auswerten der ersten Messdaten und der zweiten Messdaten technische Eigenschaften und/oder Begrenzungen der Orthese oder Prothese und/oder Bewegungsumfänge und/oder Limitierungen des Menschen berücksichtigt. Kann beispielsweise ein verwendetes Prothesenkniegelenk keine Standphasenbeugung ermöglichen, wird dies beim Auswerten der Messdaten berücksichtigt. Es ist in diesem Fall natürlich nicht sinnvoll, durch eine Korrekturmaßnahme zu versuchen, eine Standphasenbeugung zu erreichen. Wird sie dennoch als vorteilhaft und/oder notwendig angesehen, beinhaltet die Korrekturmaßnahme stattdessen den Austausch des Kniegelenks. Auch Bewegungseinschränkungen, beispielsweise eingeschränkte Bewegungsumfänge, des Menschen werden bevorzugt berücksichtigt um durch das Verfahren einen möglichst optimalen und individuell bestimmten Aufbau der Prothese oder Orthese zu erreichen. Diese Daten werden vorzugsweise in einer Datenbank hinterlegt und können beispielsweise manuell eingegeben werden. Es versteht sich, dass die elektronische Datenverarbeitungseinrichtung auf diese Datenbank Zugriff hat.

Derartige Begrenzungen der verwendeten Bauteile oder des Patienten werden bevorzug vor dem Durchführen des Verfahrens abgefragt und in einer Datenbank hinterlegt, auf die die elektronische Datenverarbeitungseinrichtung Zugriff hat. Alternativ dazu können sie auch durch Datenübermittlung, beispielsweise von einem Speichermedium oder online eingespielt und gespeichert werden.

Es ist von Vorteil, wenn ein biomechanisches Modell des Menschen mit Orthese oder Prothese hinterlegt und anhand der ersten Messdaten und/oder der zweiten Messdaten parametriert wird, das dann verwendet wird, um aus kinematischen Daten kinetische Daten zu berechnen.

Vorteilhafterweise stammen die ersten Messdaten und/oder die zweiten Messdaten zumindest auch von Sensoren, die an einer unversorgten Extremität des Menschen befestigt sind. Vorzugsweise wird ein Teil der gesunden, also unversorgten, Körpersegmenten des Menschen befestigt, während ein anderer Teil an versorgten Körpersegmenten, also an der Prothese selbst befestigt wird. Vorzugsweise wird aus den ersten Messdaten und den zweiten Messdaten eine Symmetrie des Gangbildes ermittelt. Ein Sensor, der an einer Prothese angeordnet wird gilt im Rahmen der vorliegenden Erfindung auch als an einem Körpersegment des Menschen befestigt. Unter einem Körpersegment wird vorliegend sowohl ein natürliches Körperteil eines Menschen als auch ein Bauteil einer Orthese oder Prothese verstanden, die der Mensch trägt.

Geeignete Sensoren sind beispielsweise ein Winkelsensor, Inertialsensor, Drucksensor, Kraft- und/oder Momentensensor, Video- oder Bildsensor. Durch die Kombination von Daten unterschiedlicher Sensoren und/oder unterschiedlicher Bewegungszustände werden neue Erkenntnisse gewonnen, die ohne diese Kombination nicht erhalten werden können. Die Daten unterschiedlicher Quellen werden vorzugsweise fusioniert.

Auf diese Weise können Messdaten sowohl der versorgten als auch der unversorgten Extremität aufgenommen und miteinander verglichen werden. Dies ist insbesondere für Symmetriebetrachtungen und Symmetriemessungen von großem Vorteil. Dies betrifft beispielsweise die Messung der Schrittlänge oder der Doppelschrittlänge sowie der Schrittdauer oder der Doppelschrittdauer. Auch die Ganggeschwindigkeit, der maximale Kniewinkel während der Standphase oder der maximale Kniewinkel während der Schwungphase können auf diese Weise für beide Extremitäten ermittelt und auf Symmetrien miteinander verglichen werden. Sollten hier Asymmetrien erkannt werden, kann dies ein Zeichen für entsprechende Korrekturmaßnahmen sein. Weitere Größen, die durch den wenigstens einen Sensor ermittelt werden können, sind beispielsweise der Zirkumduktionstyp, der vom Patienten ausgeführt wird, die maximale Bodenfreiheit in der Schwungphase eines Schrittes, der minimale Kniewinkel in der Standphase, sowie der prozentuale Anteil der Standphase und/oder der Schwungphase am Gangzyklus, wobei insbesondere die zeitliche Dauer betrachtet wird. Selbstverständlich können auch Kadenz, Streckenlänge und andere Parameter, wie beispielsweise Kniewinkelgeschwindigkeiten, ermittelt werden. Während der Auswertung der Messdaten können aus so ermittelten ursprünglichen Messdaten, die direkt von den Sensoren stammen, weitere abgeleitete Größen, insbesondere zeitliche Ableitungen oder Relativwinkel, ermittelt werden. Alle diese Daten und Messdaten werden vorzugsweise der Auswertung zugrunde gelegt, um einen möglichst optimalen Prothesenaufbau zu gewährleisten.

Vorzugsweise ist der erste Bewegungszustand oder der zweite Bewegungszustand ein Stehen, ein langsames Gehen, ein schnelles Gehen, Treppensteigen aufwärts oder abwärts, das Gehen entlang einer Neigung aufwärts oder abwärts, ein Stehen oder ein Sitzen, ein Aufstehen oder Hinsetzen oder ein Trippeln auf der Stelle (wechselseitiges Heben der Beine). Auch das Beschleunigen ist ein derartiger Bewegungszustand. Vorteilhafterweise unterscheiden sich der erste Bewegungszustand und der zweite Bewegungszustand auch oder nur durch eine Bodenbeschaffenheit. Dabei wird die Bewegung des ersten Bewegungszustandes, beispielsweise Gehen auf der Ebene, auf einem anderen Bodenbelag ausgeführt als die Bewegung des zweiten Bewegungszustandes. Die Bewegungen können beispielsweise auf harten Böden, beispielsweise Stein, Beton oder Holz, oder auf weichen Böden, beispielsweise Sand, Waldboden, Rasen oder Teppichboden durchgeführt werden.

Beim Auswerten der ersten Messdaten und der zweiten Messdaten werden diese vorzugsweise mit hinterlegten Referenzdaten verglichen, die vorzugsweise in einem elektronischen Datenspeicher hinterlegt sind. Dabei kann es sich um Daten anderer Menschen, beispielsweise andere Patienten mit der gleichen Erkrankung oder Behinderung und/oder der gleichen Orthese oder Prothese handeln.

Für ein solches Verfahren wird vorzugsweise ein System mit mindestens einem Sensor zum Befestigen an einem Körpersegment eines Menschen und einer elektronischen Datenverarbeitungseinrichtung verwendet, die eingerichtet ist zum Durchführen eines hier beschriebenen Verfahrens, wobei das System vorzugsweise eine Kommunikationseinrichtung aufweist, die insbesondere als Display ausgebildet ist. Auch hier gilt, dass ein Sensor, der an einer Prothese oder einem Bauteil der Prothese angeordnet ist, als ein Sensor an einem Körpersegment des Menschen gilt.

Die elektronische Datenverarbeitungseinrichtung ist vorzugsweise eingerichtet, selbstständig einen geeigneten Funktionstest zu ermitteln und durchzuführen. Bekannte Tests sind beispielsweise ein "Timed up and go test (TUG)", ein "functional reach test (FRT)", eine "2 minutes walk test (2MWT)" oder ein "four square step test (FSST)".

Aus Messdaten, die beim Gehen, das dann den ersten Bewegungszustand bildet, und beim Trippeln, das den zweiten Bewegungszustand bildet, aufgenommen wurden, lassen sich die Parallelität der Füße (Position der Füße relativ zueinander) sowie die Schrittlängensymmetrie und die Symmetrie der Standphasendauer bestimmen. Sind alle diese Parameter bekannt, die nicht alle aus den Daten nur eines Bewegungszustandes gewonnen werden können, lässt sich beispielsweise die Flexionskontraktur beurteilen.

Um die Schwungphasenextension und/oder die Schwungphasenflexion beurteilen zu können, werden vorzugsweise Messdaten beim langsamen Gehen (erster Bewegungszustand) und beim Beschleunigen (zweiter Bewegungszustand) aufgenommen.

Wird während eine ersten Bewegungszustandes festgestellt, dass der Mensch Fehlstellungen durch andere Bewegungen zu kompensieren versucht, kann der zweite Bewegungszustand auch in einer "entspannten" Durchführung der gleichen Bewegung, beispielsweise Gehen, bestehen.

Mit Hilfe der beigefügten Zeichnungen werden nachfolgend Ausführungsbeispiele der vorliegenden Erfindung näher erläutert. Es zeigt:
- Figur 1: - ein System mit mehreren Sensoren, die Informationen über Bewegungen, Positionen und/oder Orientierungen erfassen können, zum Durchführen eines Verfahrens nach einem ersten Ausführungsbeispiel der vorliegenden Erfindung,
- Figur 2: - die schematische Darstellung eines anderen Systems,
- Figur 3: - die schematische Darstellung eines Beins mit zwei Halterungen gemäß einem Ausführungsbeispiel der vorliegenden Erfindung,
- Figur 4: - die Darstellung einer Halterung gemäß einem Ausführungsbeispiel der vorliegenden Erfindung,
- Figur 5: - die Darstellung einer Halterung,
- Figuren 6 und 7: - schematische Details der Halterung aus Figur 5,
- Figuren 8 und 9: - schematische Darstellungen von Befestigungselementen,
- Figur 10: - die schematische Darstellung einer weiteren Halterung und
- Figur 11: die schematische Darstellung eines Verfahrens.

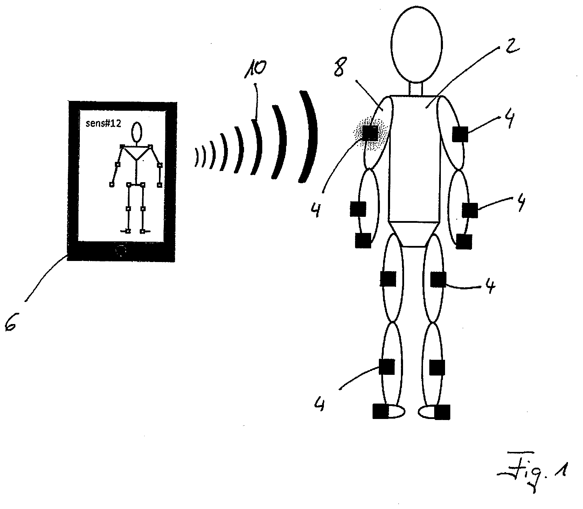

Figur 1 zeigt eine Person 2, an deren Körper eine Mehrzahl von Sensoren 4 befestigt ist. Die Sensoren 4 ermitteln Messwerte, beispielsweise Absolutwinkel, Relativwinkel, Geschwindigkeiten oder Beschleunigungen, und können diese vorteilhafterweise kabellos an eine elektronische Datenverarbeitungseinrichtung 6 übermitteln. Damit die von den Sensoren 4 ermittelten Messwerte in der elektronischen Datenverarbeitungseinrichtung 6 zutreffend ausgewertet werden können, muss zunächst jeder der Sensoren einem Körpersegment 8 zugeordnet werden. Der besseren Übersichtlichkeit wegen ist nur einer Körpersegment 8, nämlich der in Figur 1 links dargestellte Oberarm der Person 2 gekennzeichnet.

Um eine Zuordnung der Sensoren 4 zu den unterschiedlichen Körpersegmenten 8 erreichen zu können, sendet die elektronische Datenverarbeitungseinrichtung 6 im gezeigten Ausführungsbeispiel ein Signal 10, mit dem der an dem Körpersegment 8 angeordnete Sensor 4 zum Aussenden eines Sensorsignals, oder Antwortsignales, stimuliert wird.

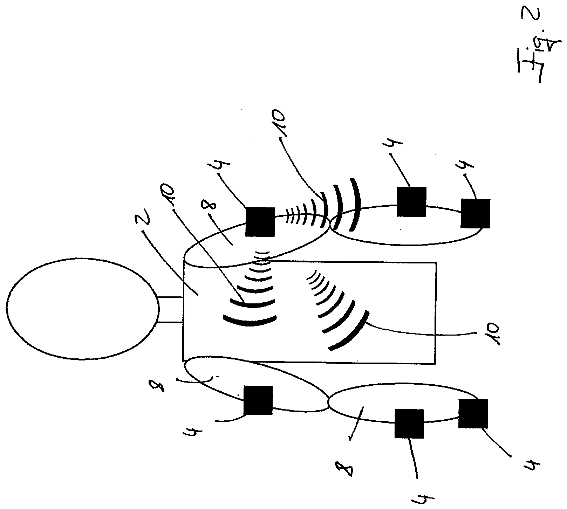

Figur 2 zeigt eine andere Ausgestaltung, bei der die Person 2 nur im Bereich der Arme über Sensoren 4 verfügt. Insbesondere die Sensoren an den Ober- und Unterarmen können durch Halterungen gemäß dem Ausführungsbeispiel der vorliegenden Erfindung, die nachfolgend näher beschrieben dargestellt sind, an den Oberarmen und Unterarmen angeordnet werden. Der in Figur 2 oben rechts am Oberarm dargestellte Sensor 4 sendet Signale 10 aus, die von den anderen Sensoren 4 detektiert werden können. Über die Bestimmung von Laufzeiten lassen sich Abstände zwischen den Sensoren 4 und damit durch Vergleich mit vorbestimmten, berechneten oder gemessenen Mustern die Anordnungen der einzelnen Sensoren 4 an den Körpersegmenten 8 bestimmen.

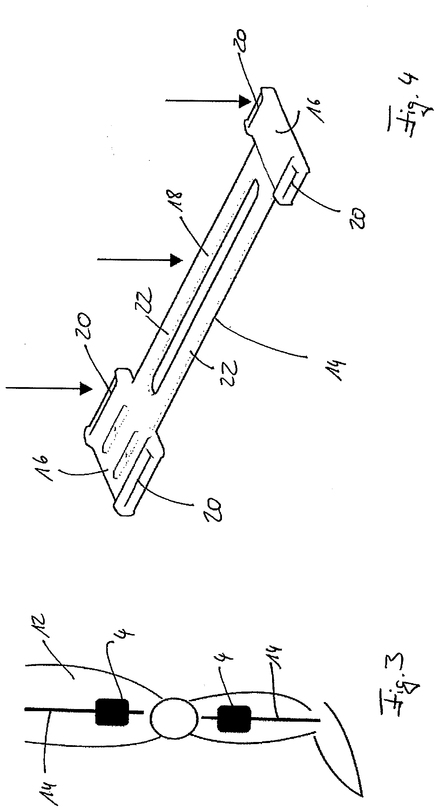

Figur 3 zeigt schematisch ein Bein 12, an dem zwei Sensoren 4 angeordnet sind. Dies geschieht über Halterungen 14, die erfindungsgemäß ausgestaltet sein können und in Figur 3 nur schematisch dargestellt sind.

Figur 4 zeigt eine solche Halterung 14. Sie verfügt über zwei Befestigungselemente 16, die durch ein Abstandselement 18 voneinander getrennt werden. Die beiden Befestigungselemente 16 verfügen über Schlitze 20, durch die beispielsweise ein Befestigungsgurt durchgeführt werden kann, so dass die Halterung 14 am jeweiligen Körpersegment 8 befestigbar ist.

Das Abstandselement 18 verfügt im gezeigten Ausführungsbeispiel über zwei Einzelelemente 22, die nebeneinander angeordnet sind. Dadurch wird erreicht, dass die Biegesteifigkeit des Abstandselementes 18 in einer ersten Richtung deutlich kleiner ist als die Biegesteifigkeit in einer zweiten Richtung.

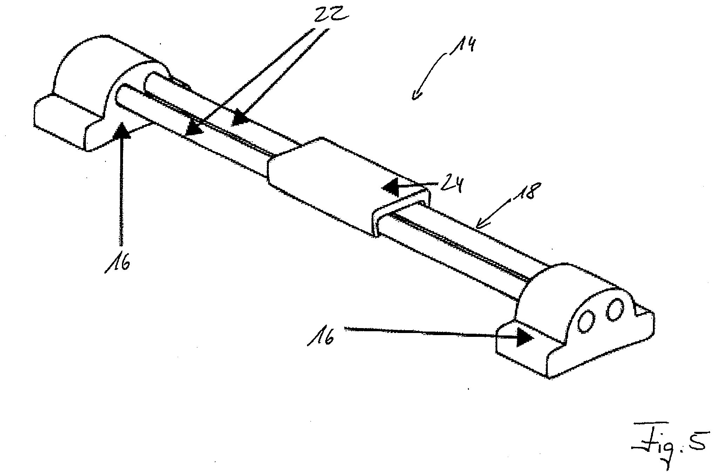

Figur 5 zeigt eine andere Ausgestaltung der Halterung 14. Auch sie verfügt über zwei Befestigungselemente 16, zwischen denen sich das Abstandselement 18 befindet, das wieder über zwei Einzelelemente 22 verfügt. An diesen Einzelelementen 22 befindet sich ein Sensorbefestigungselement 24, das im gezeigten Ausführungsbeispiel entlang der Einzelelemente 22 und damit entlang des Abstandselementes 18 verschiebbar ausgebildet ist.

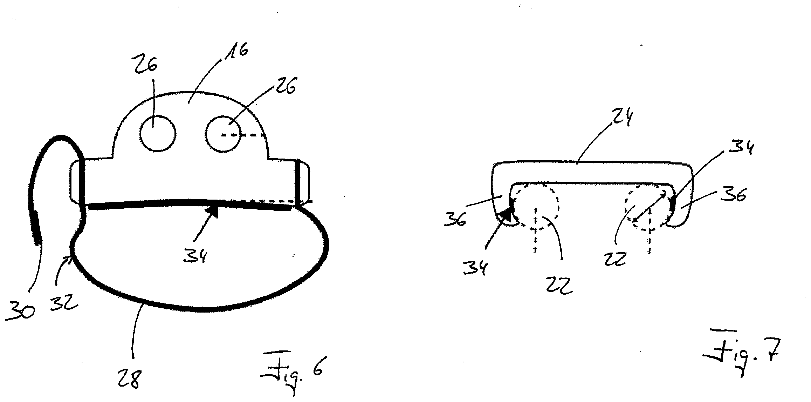

Figur 6 zeigt eine vergrößerte Darstellung aus einer anderen Perspektive eines der Befestigungselemente 16. Man erkennt zwei Öffnungen 26, in die die Einzelelemente 22 eingesteckt sind. Am Befestigungselement 16 befindet sich ein Gurt 28, der um ein Körperteil herumgelegt werden kann. Am freien Ende des Gurtes 28 befindet sich im gezeigten Ausführungsbeispiel ein Klettverschlusselement 30, das an einer Außenseite 32 des Gurtes 28 festgelegt werden kann, so dass der Gurt 28 geschlossen und das Befestigungselement 16 am Körpersegment 8 angeordnet wird. An einer dem Körpersegment 8 zugewandten Seite des Befestigungselementes 16 befindet sich im gezeigten Ausführungsbeispiel eine rutschhemmende Beschichtung 34, durch die ein Verrutschen des Befestigungselementes 16 und damit der Halterung 14 relativ zum Körpersegment 8 verhindert werden soll.

Figur 7 zeigt das Sensorbefestigungselement 24 und die nur gestrichelt dargestellten Einzelelemente 22. Das Sensorbefestigungselement 24 verfügt über zwei Klemmarme 36, die die Einzelelemente 22 wie dargestellt zumindest teilweise umgreifen und so das Sensorbefestigungselement 24 an den Einzelelementen 22 befestigen. Zwischen den Einzelelementen 22 und den Klemmarmen 36 des Sensorbefestigungselementes 24 befindet sich im gezeigten Ausführungsbeispiel eine rutschhemmende Beschichtung 34, durch die ein versehentliches Verschieben und Verrutschen des Sensorbefestigungselementes 24 relativ zum Abstandselement 18 und dessen Einzelelementen 22 verhindert wird.

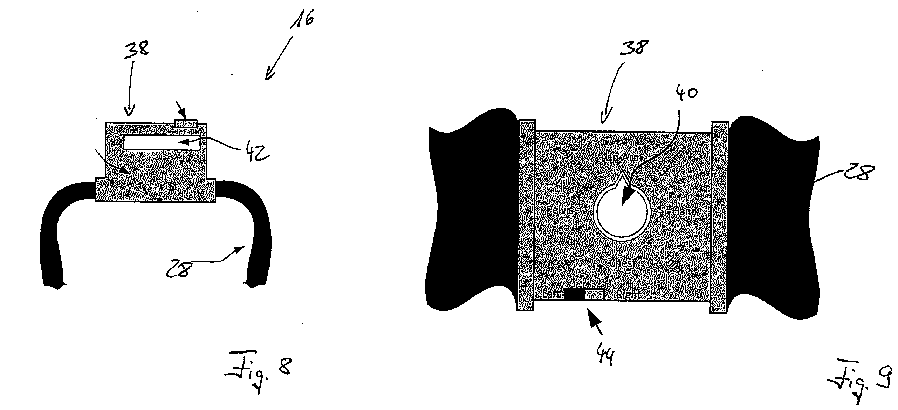

Die Figuren 8 und 9 zeigen ein Befestigungselement 16 in Form eines Gurtes 28. An ihm befindet sich eine Einstelleinrichtung 38, die über einen Drehregler 40 verfügt. Der Drehregler kann je nach Körpersegment 8, an dem ein Sensor mit diesem Befestigungselement 16 angeordnet wird, einstellen. Die entsprechende Kennung wird auf einem kleinen Display 42 dargestellt. über einen Schieberegler 44 lässt sich im gezeigten Ausführungsbeispiel zudem einstellen, ob ein Sensor 4, der an einer Halterung 14 angeordnet wird, die mit einem derartigen Befestigungselement 16 ausgerüstet ist, an einer rechten oder linken Körperseite angeordnet ist.

Durch die Einstellung des Drehreglers 40 und des Schiebereglers 44 lässt sich beispielsweise ein Signal, das vom jeweiligen Sensor 4 auf ein Aufforderungssignal 10, wie es beispielsweise in Figur 1 schematisch dargestellt ist, aussendet, einstellen. Auf diese Weise können identische Sensoren für beispielsweise unterschiedliche Patienten an unterschiedlichen Orten und Körpersegmenten 8 verwendet werden, ohne dass die Sensoren oder die elektronische Datenverarbeitungseinrichtung eingestellt werden müssen.

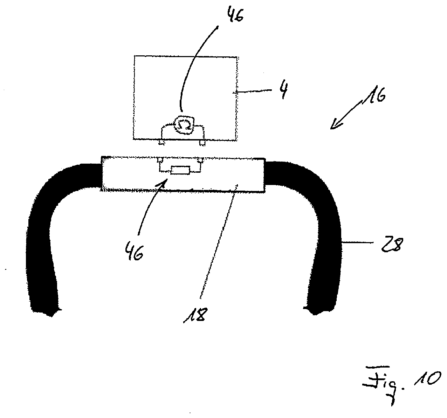

Figur 10 zeigt eine weitere Ausgestaltung eines Befestigungselementes 16, das wieder über einen Gurt 28 verfügt. Zudem ist in einer Seitenansicht das Abstandselement 18 dargestellt, das im gezeigten Ausführungsbeispiel so ausgebildet ist, dass der Sensor 4 direkt am Abstandselement 18 angeordnet werden kann. Dabei verfügt sowohl das Abstandselemente 18 als auch der Sensor 4 über eine elektronische Baugruppe 46, die durch Anordnen des Sensors 4 am Abstandselement 18 in elektrischen Kontakt gebracht werden. Auf diese Weise wird ein Signal, das der Sensor 4 zu einer elektronischen Datenverarbeitungseinrichtung 6 aussendet, modifiziert, so dass die elektronische Datenverarbeitungseinrichtung den Signalinformationen über die Art des Sensors 4 und/oder die Position und Orientierung des Sensors 4, also insbesondere das Körpersegment 8 entnehmen kann.

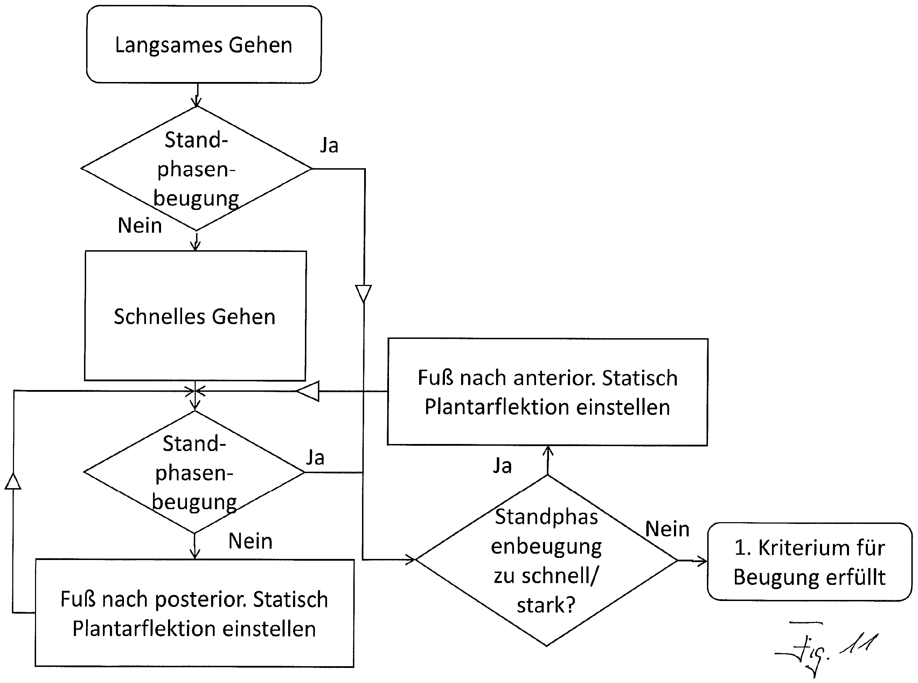

Figur 11 zeigt den schematischen Ablauf eines zum Einrichten einer Prothese oder Orthese oder zum Bestimmen von Fehlstellungen im Aufbau einer Prothese oder Orthese. Im vorliegenden Verfahren soll geprüft werden, ob ein Prothesenfuß in anterior-posteriore Richtung richtig positioniert ist oder ob er in anteriorer oder posteriorer Richtung verschoben werden muss. Dazu werden im ersten Verfahrensschritt, der mit "langsames Gehen" gekennzeichnet ist, die ersten Messdaten aufgenommen. Dabei handelt es sich vorzugsweise um den Kniewinkel, also dem Winkel zwischen dem Oberschenkel und dem Unterschenkel. Dies kann beispielsweise über zwei Inertialwinkelsensoren, die den Winkel des Oberschenkels relativ zur vertikalen und den Winkel des Unterschenkels relativ zur vertikalen messen, geschehen. Die Vertikale ist dabei die Richtung entlang der Schwerkraft. Anschließend werden die so aufgenommenen ersten Messdaten dahingehend ausgewertet, ob eine Standphasenbeugung vorliegt. Sofern dies nicht der Fall ist, werden die zweiten Messdaten in einem weiteren Verfahrensschritt, der mit "schnelles Gehen" gekennzeichnet ist, aufgenommen. Das schnelle Gehen bildet dabei den zweiten Bewegungszustand. Langsames Gehen und schnelle Gehen sind dabei zunächst nicht auf bestimmte Geschwindigkeitsbereiche des Gehens eingeschränkt. Durch die Bezeichnungen wird jeweils lediglich dargestellt, dass der erste Bewegungszustand " langsames Gehen" eine kleinere Vorwärtsgeschwindigkeit aufweist als der zweite Bewegungszustand "schnelles Gehen". Das Gehen selbst findet dabei auf einer Ebene statt, die vorzugsweise senkrecht auf der Vertikalen, also entlang der Horizontalen, steht. Eine Neigung weist diese Ebene vorteilhafterweise nicht auf.

Auch die so aufgenommenen zweiten Messdaten werden daraufhin untersucht, ob eine Standphasenbeugung vorliegt. Bei einer Standphasenbeugung handelt es sich um eine Beugung des Knies während der ersten Hälfte der Standphase eines Gangzyklus. Dabei kommt es nach dem Fersenauftritt zu einer kurzfristigen Verringerung des Kniewinkels, also zu einem Einbeugen des Knies. Sofern dies in beiden Bewegungszuständen, also in beiden aufgenommenen Messdaten nicht vorhanden ist, wird vorgeschlagen, den Prothesenfuß in posteriore Richtung zu verschieben. Anschließend sollte in einem statischen Aufbau die Plantarflexion eingestellt werden. Kann jedoch aus den ersten Messdaten und/oder den zweiten Messdaten eine Standphasenbeugung detektiert werden, muss überprüft werden, ob diese in einem angemessenen Geschwindigkeitsbereich stattfindet und eine ausreichende Stärke aufweist. Eine zu schnelle Standphasenbeugung oder eine zu starke Standphasenbeugung sind ebenfalls auf Fehlstellungen im Prothesenaufbau zurückzuführen, die korrigiert werden müssen. Um dies zu tun wird vorgeschlagen, den Fuß nach anterior zu verschieben und anschließend vorzugsweise die Plantarflexion statisch einzustellen. Dieses Verfahren wird solange durchlaufen, bis das Beugungskriterium erfüllt ist und die anterior-posteriore Ausrichtung des Prothesenfusses ausreichend bestimmt ist. Dieses Verfahren wird vorzugsweise bei transtibial amputierten Patienten, die folglich ein natürliches Knie aufweisen, durchgeführt.

### Bezugszeichenliste

- 2: Person
- 4: Sensor
- 6: elektronische Datenverarbeitungseinrichtung
- 8: Körpersegment
- 10: Signal
- 12: Bein
- 14: Halterung
- 16: Befestigungselement
- 18: Abstandselement
- 20: Schlitz
- 22: Einzelelement

- 24: Sensorbefestigungselement
- 26: Öffnung
- 28: Gurt
- 30: Klettverschlusselement
- 32: Außenseite
- 34: rutschhemmende Beschichtung
- 36: Klemmarm
- 38: Einstelleinrichtung
- 40: Drehregler
- 42: Display
- 44: Schieberegler
- 46: elektronische Baugruppe

## Patentansprüche

1. Verfahren, bei dem
a. wenigstens zwei Sensoren (4) an unterschiedlichen Körpersegmenten (8) eines Menschen befestigt werden,
b. die wenigstens zwei Sensoren (4) Signale, die Messdaten und eine individuelle Sensorkennung enthalten, an eine elektronische Datenverarbeitungseinrichtung (6) senden und
c. die elektronische Datenverarbeitungseinrichtung (6) die Körpersegmente (8) bestimmt, an denen die Sensoren (4) angeordnet sind oder anzuordnen sind und die Messdaten der wenigstens zwei Sensoren (4) den Körpersegmenten (8) zuordnet, **dadurch gekennzeichnet, dass**
die Sensoren (4) eingerichtet sind, Informationen über Abstände zwischen jeweils zwei Sensoren (4) zu ermitteln und diese in den gesendeten Signalen an die elektronische Datenverarbeitungseinrichtung (6) zu senden.

2. Verfahren nach Anspruch 1, **dadurch gekennzeichnet, dass** der elektronischen Datenverarbeitungseinrichtung (6) wenigstens ein Detektor zugeordnet ist, der zumindest einen Teil der Signale der Sensoren (4) detektiert, wobei die elektronische Datenverarbeitungseinrichtung (6) aus den detektierten Signalen, insbesondere aus einer Richtung oder Lage, aus der die individuelle Senderkennung gesendet wird oder aus einem zeitlichen Verlauf der Messdaten, das Körpersegment (8) bestimmt.

3. Verfahren nach Anspruch 1 oder 2, **dadurch gekennzeichnet, dass** die Signale der Sensoren (4) zusätzlich Informationen über das Körpersegment (8) enthalten.

4. Verfahren nach Anspruch 3, **dadurch gekennzeichnet, dass** die Sensoren (4) mittels einer Halterung (14) an dem Körpersegment (8) befestigt sind, die vom Sensor (4) gesendeten Signale beeinflussen.

5. Verfahren nach Anspruch 4, **dadurch gekennzeichnet, dass** die Sensoren (4) die Informationen über das Körpersegment (8) der Halterung (14) entnimmt.

6. Verfahren nach Anspruch 4 oder 5, **dadurch gekennzeichnet, dass** die Halterung (14) das Körpersegment (8) ermittelt, an dem sie befestigt ist.

7. Verfahren nach einem der Ansprüche 1 bis 4, **dadurch gekennzeichnet, dass** die Sensoren über eine Einstelleinrichtung verfügen, durch die die Information über das Körpersegment (8) eingestellt werden.

8. Verfahren nach einem der vorstehenden Ansprüche, **dadurch gekennzeichnet, dass** die wenigstens zwei Sensoren (4) die Signale nur an die elektronische Datenverarbeitungseinrichtung (6) senden, wenn sie ein Abfragesignal erhalten haben.

9. Verfahren nach einem der vorstehenden Ansprüche, **dadurch gekennzeichnet, dass** die Sensoren (4) Testsignale aussenden, die von anderen Sensoren (4) detektiert und Informationen über die so detektierten Signale, insbesondere Detektionszeitpunkte, von den Sensoren (4) an die elektronische Datenverarbeitungseinrichtung (6) übermittelt werden.

10. Verfahren , bei dem
a. wenigstens zwei Sensoren (4) an unterschiedlichen Körpersegmenten (8) eines Menschen befestigt werden,
b. die wenigstens zwei Sensoren (4) Signale, die Messdaten enthalten, an eine elektronische Datenverarbeitungseinrichtung (6) senden und
c. die elektronische Datenverarbeitungseinrichtung (6) ein Steuersignal an die wenigstens zwei Sensoren (4) aussendet und die wenigstens zwei Sensoren (4) als Reaktion auf das Steuersignal ein von einem Menschen detektierbares Antwortsignal aussenden,
**dadurch gekennzeichnet, dass** die Sensoren (4) eingerichtet sind, Informationen über Abstände zwischen jeweils zwei Sensoren (4) zu ermitteln und diese in den gesendeten Signalen an die elektronische Datenverarbeitungseinrichtung (6) zu senden.

11. System mit wenigstens zwei Sensoren (4) und einer elektronische Datenverarbeitungseinrichtung (6), das eingerichtet ist zum Durchführen eines Verfahrens nach einem der vorstehenden Ansprüche.

## Claims

1. A method in which
a. at least two sensors (4) are fixed to different body parts (8) of a person,
b. the at least two sensors (4) send signals, which contain measurement data and an individual sensor identifier, to an electronic data processing device (6), and
c. the electronic data processing device (6) determines the body parts (8) on which the sensors (4) are arranged or are to be arranged and allocates the measurement data of the at least two sensors (4) to the body parts (8) **characterized by** the fact that the sensors (4) are configured to detect information on distances between two sensors (4) and to send said information in the signals sent to the electronic data processing device (6).

2. The method according to claim 1, **characterized by** the fact that at least one detector is allocated to the electronic data processing device (6), said detector detecting at least one part of the signals from the sensors (4), wherein the electronic data processing device (6) determines the body part (8) using the detected signals, especially a direction or position from which the individual identifier is sent, or from a time path of the measurement data.

3. The method according to claim 1 or 2, **characterized by** the fact that the signals of the sensors (4) also contain information on the body part (8).

4. The method according to claim 3, **characterized by** the fact that the sensors (4) are fixed to the body part (8) by means of a bracket (14), which has an effect on signals sent by the sensors (4).

5. The method according to claim 4, **characterized by** the fact that the sensors (4) take the information on the body part (8) from the bracket (14).

6. The method according to claim 4 or 5, **characterized by** the fact that the bracket (14) detects the body part (8) to which it is fixed.

7. The method according to one of the claims 1 to 4, **characterized by** the fact that the sensors feature an adjustment device, by way of which the information on the body part (8) can be adjusted.

8. The method according to one of the above claims, **characterized by** the fact that the at least two sensors (4) only send the signals to the electronic data processing device (6) if they have received a request signal.

9. The method according to one of the above claims, **characterized by** the fact that the sensors (4) emit test signals that are detected by other sensors (4), and information on the signals detected in this way, especially the times of detection, are transmitted from the sensors (4) to the electronic data processing device (6).

10. A method in which
a. at least two sensors (4) are fixed to different body parts (8) of a person,
b. the at least two sensors (4) send signals, which contain measurement data, to an electronic data processing device (6), and
c. the electronic processing device (6) sends a control signal to the at least two sensors (4) and the at least two sensors (4), in response to the control signal, emit a response signal that can be detected by a person, **characterized in that** the sensors (4) are configured to detect information on distances between two sensors (4) and to send said information in the signals sent to the electronic data processing device (6).

11. A system with at least two sensors (4) and an electronic data processing device (6) that is configured to conduct a method according to one of the above claims.

## Revendications

1. Procédé dans lequel
a. au moins deux capteurs (4) sont fixés à différents segments corporels (8) d'un être humain,
b. lesdits au moins deux capteurs (4) envoient des signaux, contenant des données de mesure et un identifiant de capteur individuel, à un dispositif électronique de traitement de données (6), et
c. le dispositif électronique de traitement de données (6) détermine les segments corporels (8) sur lesquels les capteurs (4) sont disposés ou doivent être disposés, et associe les données de mesure desdits au moins deux capteurs (4) aux segments corporels (8),
**caractérisé en ce que**
les capteurs (4) sont conçus pour déterminer des informations relatives à des distances entre deux capteurs (4) respectifs et pour les envoyer dans les signaux envoyés au dispositif électrique de traitement de données (6).

2. Procédé selon la revendication 1, **caractérisé en ce qu'**au dispositif électronique de traitement de données (6) est associé au moins un détecteur qui détecte au moins une partie des signaux des capteurs (4), le dispositif électronique de traitement de données (6) déterminant le segment corporel (8) à partir des signaux détectés, en particulier à partir d'une direction ou d'une position depuis laquelle l'identifiant individuel de l'émetteur est envoyé, ou à partir d'une évolution dans le temps des données de mesure.

3. Procédé selon la revendication 1 ou 2, **caractérisé en ce que** les signaux des capteurs (4) contiennent en supplément des informations relatives au segment corporel (8).

4. Procédé selon la revendication 3, **caractérisé en ce que** les capteurs (4) sont fixés au segment corporel (8) au moyen d'un support (14) qui influence les signaux envoyés par le capteur (4).

5. Procédé selon la revendication 4, **caractérisé en ce que** les capteurs (4) prélèvent dans le support (14) les informations relatives au segment corporel (8).

6. Procédé selon la revendication 4 ou 5, **caractérisé en ce que** le support (14) détermine le segment corporel (8) auquel il est fixé.

7. Procédé selon l'une des revendications 1 à 4, **caractérisé en ce que** les capteurs disposent d'un dispositif de réglage par lequel les informations relatives au segment corporel (8) sont réglées.

8. Procédé selon l'une des revendications précédentes, **caractérisé en ce que** lesdits au moins deux capteurs (4) n'envoient les signaux au dispositif électronique de traitement de données (6) que lorsqu'ils ont reçu un signal d'interrogation.

9. Procédé selon l'une des revendications précédentes, **caractérisé en ce que** les capteurs (4) émettent des signaux de test qui sont détectés par d'autres capteurs (4), et les informations relatives aux signaux ainsi détectés, en particulier aux instants de détection, sont transmises par les capteurs (4) au dispositif électronique de traitement de données (6).

10. Procédé dans lequel
a. au moins deux capteurs (4) sont fixés à différents segments corporels (8) d'un être humain,
b. lesdits au moins deux capteurs (4) envoient des signaux contenant des données de mesure à un dispositif électronique de traitement de données (6), et
c. le dispositif électronique de traitement de données (6) émet un signal de commande auxdits au moins deux capteurs (4), et lesdits au moins deux capteurs (4) émettent, en réponse au signal de commande, un signal de réponse détectable par un être humain,
**caractérisé en ce que**
les capteurs (4) sont conçus pour déterminer des informations relatives à des distances entre deux capteurs (4) respectifs et pour les envoyer dans les signaux envoyés au dispositif électrique de traitement de données (6).

11. Système comprenant au moins deux capteurs (4) et un dispositif électronique de traitement de données (6), qui est conçu pour mettre en oeuvre un procédé selon l'une des revendications précédentes.
